# EUROPÄISCHE PATENTSCHRIFT

(11) **EP 3 017 283 B1**
(45) Veröffentlichungstag und Bekanntmachung des Hinweises auf die Patenterteilung: **25.10.2017**
(21) Anmeldenummer: 14735853.5
(22) Anmeldetag: 27.06.2014
(51) Int. Cl.: G01K 13/00, A61B 5/01, G01K 15/00

(54) **MESSVORRICHTUNG ZUR MESSUNG EINER KÖRPERFUNKTION**
MEASURING DEVICE FOR MEASURING BODY FUNCTIONS
DISPOSITIF DE MESURE D'UNE FONCTION CORPORELLE

(30) Priorität: 03.07.2013 DE 102013011141
(43) Veröffentlichungstag der Anmeldung: 11.05.2016
(73) Patentinhaber: Drägerwerk AG & Co. KGaA, 23558 Lübeck (DE)
(72) Erfinder: KOCH, Jochim, 23909 Ratzeburg (DE); GRASSL, Thomas, 23560 Lübeck (DE)
(74) Vertreter: Mildner, Volker
(86) Internationale Anmeldenummer: PCT/EP2014/001764
(87) Internationale Veröffentlichungsnummer: WO 2015/000570

(56) Entgegenhaltungen:
- GB-A- 2 061 496
- US-A- 4 331 161
- US-A1- 2006 270 942
- US-B1- 6 526 300

## Beschreibung

Die Erfindung betrifft eine Messvorrichtung zur Messung einer Körperfunktion mittels zumindest eines mit einer Haut eines Menschen oder Patienten mittelbar oder unmittelbar in Berührung stehenden Sensors und ein Verfahren zum Betrieb einer solchen Messvorrichtung. Die Messung der Körperfunktion bezieht sich dabei zum Beispiel auf eine Körpertemperatur des Patienten.

Derartige Messvorrichtungen, speziell solche Messvorrichtungen in Form einer Temperaturmessvorrichtung, sind an sich bekannt, zum Beispiel aus der DE 10 2005 037 921 B3. Problematisch bei Messvorrichtungen, deren Anbringung auf der Hautoberfläche des Patienten vorgesehen ist, ist jedoch, dass sich die jeweilige Messvorrichtung lösen kann und damit keine verlässliche Messung der Körperfunktion mehr möglich ist. Als besonders problematisch haben sich dabei Situationen herausgestellt, bei denen sich die Messvorrichtung noch auf der Hautoberfläche befindet, aber kein für ein verlässliches Messergebnis ausreichender Kontakt mit der Hautoberfläche mehr besteht. Als Beispiel kann insoweit die Messung einer Körpertemperatur eines Neugeborenen in einem Inkubator genannt werden, wobei die Innentemperatur des Inkubators mit Hinblick auf die gemessene Körpertemperatur geregelt wird. Wenn eine dafür vorgesehene Temperaturmessvorrichtung sich noch auf der Hautoberfläche des Neugeborenen befindet, ist durch einfachen optischen Augenschein nicht erkennbar, ob die von der Temperaturmessvorrichtung gelieferten Messwerte verlässlich sind. Wenn sich die Temperaturmessvorrichtung tatsächlich bereits teilweise von der Hautoberfläche gelöst hat, erfolgt aufgrund der Temperaturregelung anhand eines dann fehlerhaften Messwerts zum Beispiel eine unnötige Erhöhung der Innentemperatur des Inkubators. Dieses Szenario ist ausdrücklich nur ein beispielhaftes Szenario und mit anderen durch eine Messvorrichtung überwachten Körperfunktionen können andere Beispiele gebildet werden, die ebenfalls illustrieren, dass es von besonderer Wichtigkeit ist zu erkennen, ob die jeweils gelieferten Messwerte verlässlich sind, ob sich also die Messvorrichtung noch in einem solchen Maß in Kontakt mit der Hautoberfläche des Patienten befindet, dass verlässliche Messergebnisse erwartet werden können.

Die oben genannte DE 10 2005 037 921 B3 befasst sich bereits mit dieser Problematik und schlägt eine Möglichkeit vor, wie ein ungenügender Hautkontakt der Messvorrichtung erkannt werden Weitere Dokumente die Lösungen zu diesem Problem offenbaren sind die US 4,331,161 und die US 6,526,300 B1.

Eine Aufgabe der vorliegenden Erfindung besteht darin, eine weitere Ausführungsform einer Messvorrichtung der eingangs genannten Art und ein Verfahren zu deren Betrieb anzugeben, die bzw. das die Erkennung eines ungenügenden Hautkontakts der Messvorrichtung erlaubt und damit die Verlässlichkeit des jeweils erfassten Messwerts und/oder die Sicherheit des Patienten erhöht.

Erfindungsgemäß wird diese Aufgabe durch eine derartige Messvorrichtung mit den Merkmalen des Anspruchs 1 gelöst. Dazu umfasst eine solche Messvorrichtung, die mindestens einen Sensor aufweist und die zur Erfassung eines Messwerts mittels des Sensors auf der Hautoberfläche eines Patienten anbringbar ist, zumindest eine erste und zumindest eine zweite Kontaktfläche auf einer zum Kontakt mit der Hautoberfläche des Patienten vorgesehenen Unterseite der Messvorrichtung sowie Mittel zum Erfassen eines aufgrund eines jeweils wirksamen elektrischen Widerstands zwischen der zumindest einen ersten und zweiten Kontaktfläche resultierenden Messstroms. Weiter sind die Sensorik und die beiden Kontaktflächen elektrisch parallel geschaltet und es befindet sich entweder in einem Strompfad zur Sensorik oder in einem Strompfad zu den Kontaktflächen ein Stromsperrelement, zum Beispiel eine Diode. Der erfasste Messstrom ist dabei ein Maß für einen elektrischen Widerstand oder eine elektrische Leitfähigkeit zwischen der zumindest einen ersten und zweiten Kontaktfläche - im Folgenden nur zur sprachlichen Vereinfachung kurz als erste und zweite Kontaktfläche und zusammen als "die Kontaktflächen" oder "beide Kontaktflächen" bezeichnet.

Der aufgrund eines Stromflusses zwischen den Kontaktflächen resultierende Messstrom oder ein auf dieser Basis generierbares Signal werden im Folgenden auch als Kontaktsignal bezeichnet, denn ein Messstrom kann nur fließen, wenn sich die Messvorrichtung noch in ausreichendem Kontakt mit der Hautoberfläche des Patienten befindet. Zur Unterscheidung werden ein Messstrom von dem von der Messvorrichtung umfassten Sensor oder ein auf dieser Basis generierbares Signal im Folgenden als Sensorsignal bezeichnet.

Bei einem Verfahren zum Betrieb einer solchen Messvorrichtung ist vorgesehen, dass ein aufgrund eines jeweils wirksamen elektrischen Widerstands zwischen der zumindest einen ersten und zweiten Kontaktfläche resultierender Messstrom erfasst wird und dass dieser als Kontaktsignal ausgewertet oder auf Basis des Messstroms ein Kontaktsignal generiert wird.

Ein Vorteil der Erfindung besteht darin, dass auf Basis des Kontaktsignals unmittelbar und eindeutig feststeht, ob noch ein ausreichender Hautkontakt der Messvorrichtung insgesamt gegeben ist.

Dies basiert darauf, dass der elektrische Widerstand zwischen den beiden Kontaktflächen stark ansteigt, wenn sich die Messvorrichtung von der Hautoberfläche löst. Wenn eine der Kontaktflächen nicht mehr mit der Hautoberfläche in Kontakt ist, ergibt sich ein so hoher Widerstand, dass kein Messstrom mehr fließen kann und das Kontaktsignal verschwindet.

Jeder so als unzureichend ermittelte Hautkontakt stellt eine Fehlersituation in Bezug auf die Überwachung der jeweiligen Körperfunktion dar und eine solche Fehlersituation kann dem Patienten und/oder dem medizinischen Personal angezeigt werden, damit geeignete Gegenmaßnahmen getroffen werden können, insbesondere die Messvorrichtung wieder korrekt platziert wird. Zusätzlich oder alternativ kann eine solche Fehlersituation auch einem Medizingerät, an welches die Messvorrichtung angeschlossen ist, signalisiert werden, damit dem Patienten und/oder dem medizinischen Personal mittels des Medizingeräts die Fehlersituation angezeigt werden kann und geeignete Gegenmaßnahmen getroffen werden können.

Der Widerstand oder als Maß für den Widerstand eine Leitfähigkeit oder eine Höhe eines zwischen den Kontaktflächen fließenden Stroms kann gemessen werden. Jede dafür geeignete Schaltung oder Vorrichtung wird hier und im Folgenden allgemein als Mittel zum Erfassen des Messstroms, eines elektrischen Widerstands oder einer elektrischen Leitfähigkeit zwischen den beiden Kontaktflächen bezeichnet. Eine solche Schaltung ist entsprechend auch ein Mittel zur Generierung des Kontaktsignals. Ein Beispiel für eine in dieser Hinsicht in Betracht kommende Schaltung ist eine Auswerteschaltung mit einer an sich bekannten sogenannten Darlington-Schaltung.

Mittels des Stromsperrelements sind eine Bestromung und eine Generierung, und Auswertung des Kontaktsignals und des Sensorsignals mit einer einfachen zweiadrigen Zuleitung möglich. Dies macht die Messvorrichtung durch Vermeidung zum Beispiel einer ansonsten benötigten mehr als zweiadrigen Zuleitung besonders preiswert. Dabei ist vorgesehen, dass die Messvorrichtung zum Erzeugen eines Messstroms getaktet mit einer jeweils wechselnden Polarität bestrombar ist und im Betrieb in dieser Weise bestromt wird. Wenn sich das Stromsperrelement zum Beispiel im Strompfad zu den Kontaktflächen befindet, ergibt sich die folgende Situation: Während einer ersten Taktphase fließt der zugeführte Strom zum Beispiel sowohl zur Sensorik wie auch zu den Kontaktflächen. Während einer zweiten, komplementären Taktphase fließt der zugeführte Strom aufgrund des entsprechend der Polarität dann wirksam werdenden Stromsperrelements nur zur Sensorik. Während der ersten Taktphase werden also das Kontaktsignal sowie das Sensorsignal und ein entsprechender Messstrom generiert. Aufgrund der Parallelschaltung liegen die beiden Signale am Ausgang der Messvorrichtung in Form eines Summensignals vor. Während der zweiten Taktphase ergibt sich ausschließlich ein Sensorsignal von der Sensorik. Eine einfache Umpolung des zugeführten Stroms reicht damit aus, um bei einer ersten Polarität zum Beispiel die Sensorik und die Kontaktflächen zu versorgen und bei einer zweiten, umgekehrten Polarität nur die Sensorik zu versorgen. Bei einer Auswertung des von der Messvorrichtung zurückgelieferten Messstroms entsprechend der Taktung des zugeführten Stroms ist damit das während der ersten Taktphasen jeweils gelieferte Summensignal (Kontaktsignal plus Sensorsignal) eindeutig von dem während der zweiten Taktphasen jeweils gelieferten Sensorsignal unterscheidbar. Eine eventuelle Ablösung der Messvorrichtung von der Hautoberfläche des Patienten ist auch an dem Summensignal erkennbar. Wenn nämlich das Summensignal unter einen vorgegebenen Schwellwert fällt, lässt sich dies als Ablösung der Messvorrichtung auswerten. Der Schwellwert wird dafür anhand des normalerweise von der Auswerteschaltung erwarteten Beitrags zu dem Summensignal festgelegt, so dass zum Beispiel ein Wert geringfügig unter einem solchen normalerweise erwarteten Beitrag gewählt wird.

Vorteilhafte Ausgestaltungen der Erfindung sind Gegenstand der Unteransprüche. Dabei verwendete Rückbeziehungen weisen auf die weitere Ausbildung des Gegenstands des Hauptanspruchs durch die Merkmale des jeweiligen Unteranspruchs hin und sind nicht als ein Verzicht auf die Erzielung eines selbständigen, gegenständlichen Schutzes für die Merkmalskombinationen der rückbezogenen Unteransprüche zu verstehen. Des Weiteren ist im Hinblick auf eine Auslegung der Ansprüche bei einer näheren Konkretisierung eines Merkmals in einem nachgeordneten Anspruch davon auszugehen, dass eine derartige Beschränkung in den jeweils vorangehenden Ansprüchen nicht vorhanden ist.

Bei einer Ausführungsform der Messvorrichtung ist vorgesehen, dass die Messvorrichtung neben dem bereits erwähnten Sensor zumindest noch einen weiteren, artgleichen Sensor, also zum Beispiel einen ersten und einen zweiten Temperatursensor, einen ersten und einen zweiten Drucksensor usw., umfasst. Bei einer solchen Messvorrichtung, die zum Beispiel zwei Temperatursensoren umfasst, kann gleichzeitig die Körpertemperatur des Patienten und eine der Umgebungstemperatur nahen Temperatur erfasst werden. Dann kann zum Beispiel die Körpertemperatur in Relation zur Umgebungstemperatur betrachtet werden. - Zur sprachlichen Vereinfachung wird im Folgenden der oder jeder Sensor einzeln und zusammen kurz als Sensorik bezeichnet.

Wenn bei der Messvorrichtung die Kontaktflächen in Form eines Rasters oder labyrinthisch auf der Unterseite der Messvorrichtung angeordnet sind, ergibt sich eine vergrößerte Gesamtfläche der Kontaktflächen und vor allem eine örtliche Verteilung der Kontaktflächen auf der Unterseite der Messvorrichtung. Aufgrund dieser örtlichen Verteilung wird ein Verlust des Kontakts der Messvorrichtung mit der Hautoberfläche im Bereich zum Beispiel einer Ecke der Messvorrichtung nicht als Fehlersituation ausgewertet, weil bei einem solchen Kontaktverlust eine verlässliche Ermittlung eines Messwerts in Bezug auf die jeweils überwachte Körperfunktion immer noch möglich sein kann. Eine solche Gestaltung und Anordnung der Kontaktflächen vermeidet also in vorteilhafter Weise unnötige Fehlalarme. Neben einer solchen rasterartigen oder labyrinthischen Anordnung kommt zum Beispiel eine kammartige Anordnung in Betracht, bei der die beiden Kammflächen ineinander greifen. Auch eine solche Anordnung oder jede ähnlich wirkende Anordnung soll für die Auslegung der hier vorgelegten Beschreibung als von dem Begriff "labyrinthisch" umfasst gelten.

Wenn die Messvorrichtung selbst eine in Abhängigkeit von dem jeweils generierten Kontaktsignal ansteuerbare Anzeigeeinrichtung umfasst, ist bei einer Aktivierung einer solchen Anzeigeeinrichtung unmittelbar erkennbar, bei welcher Messvorrichtung ein ungenügender Kontakt mit der Hautoberfläche des Patienten besteht. Der Begriff Anzeigeeinrichtung meint schwerpunktmäßig optische Anzeigeeinrichtungen, also zum Beispiel eine LED oder dergleichen. Für die Auslegung des Begriffs soll allerdings gelten, dass dieser auch Einrichtungen umfasst, die auf akustischer Basis einen solchen ungenügenden Hautkontakt signalisieren können und damit ebenfalls als Anzeigeeinrichtung fungieren. Ein Beispiel für eine solche Anzeigeeinrichtung ist ein elektrischer Summer.

Bei einer besonderen Ausführungsform der hier und im Folgenden beschriebenen Messvorrichtung ist vorgesehen, dass diese eine lokale Energiequelle zur Energieversorgung zumindest der Anzeigeeinrichtung oder eines ASICs oder dergleichen mit zumindest einer Anzeigeeinrichtung aufweist. Als lokale Energiequelle kommen ein Kondensator und/oder ein Thermoelement oder ein Peltier-Element in Betracht. Ein als lokale Energiequelle fungierender Kondensator kann im Rahmen der elektrischen Versorgung (Bestromung) der Messvorrichtung geladen werden. Mittels eines Thermoelements oder eines Peltier-Elements kann eine lokale Temperaturdifferenz, zum Beispiel eine Differenz zwischen einer Temperatur auf der Hautoberfläche und einer Umgebungstemperatur, zum Aufladen der lokalen Energiequelle und damit für die Energieversorgung der Anzeigeeinrichtung, des ASICs usw. ausgenutzt werden.

Bei einer weiteren besonderen Ausführungsform der hier und im Folgenden beschriebenen Messvorrichtung ist vorgesehen, dass diese auf einer zum Kontakt mit der Hautoberfläche des Patienten vorgesehenen Unterseite eine Haftschicht umfasst, die geeignet ist, eine Haftung der Messvorrichtung auf der Hautoberfläche des Patienten aufgrund von Van-der-Waalschen Kräften zu bewirken, und dass die Kontaktflächen an einer zum Kontakt mit der Hautoberfläche des Patienten vorgesehenen Oberfläche der Haftschicht in diese eingebettet sind oder auf dieser aufgebracht sind. Eine solche Haftschicht mit darin eingebetteten Kontakten kann heute in Form einer dünnen Folie hergestellt werden. Weitere Einzelheiten zu einer solchen Folie finden sich zum Beispiel in dem Fachartikel "Epidermal electronics" von Dae-Hyeong Kim et.al. (Science 12 August 2011: Vol. 333 no. 6244 pp. 840-843).

Insgesamt ist die Erfindung auch ein System mit einem Medizingerät und zumindest einer mittels einer ersten Zuleitung und einer zweiten Zuleitung an das Medizingerät angeschlossenen Messvorrichtung der hier und im Folgenden beschriebenen Art. Der Messvorrichtung ist dabei mittels des Medizingeräts in der oben beschriebenen Art und Weise alternierend über die erste und die zweite Zuleitung ein Speisestrom zuführbar und im Betrieb des Systems wird der Messvorrichtung mittels des Medizingeräts auf diese Weise der Speisestrom zugeführt. Über eine jeweils komplementäre Zuleitung ist dem Medizingerät durch die Messvorrichtung der Messstrom und damit das Kontaktsignal und das Sensorsignal zuführbar und im Betrieb des Systems wird dem Medizingerät durch die Messvorrichtung der Messstrom zugeführt. Bei einer Zuführung des Speisestroms zur Messvorrichtung über die erste Zuleitung ist die komplementäre Zuleitung, über die dem Medizingerät der Messstrom zugeführt wird, die zweite Zuleitung und umgekehrt. Der Messstrom ist mittels des Medizingeräts auswertbar und wird im Betrieb des Systems durch das Medizingerät ausgewertet. Eine solche Auswertung umfasst zum Beispiel, dass aufgrund des Kontaktsignals eine optische oder akustische Anzeigevorrichtung auf Seiten des Medizingeräts angesteuert wird.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen.

Das oder jedes Ausführungsbeispiel ist nicht als Einschränkung der Erfindung zu verstehen. Vielmehr sind im Rahmen der vorliegenden Offenbarung Abänderungen und Modifikationen möglich, insbesondere solche Varianten und Kombinationen, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen für den Fachmann im Hinblick auf die Lösung der Aufgabe entnehmbar sind und durch kombinierbare Merkmale zu einem neuen Gegenstand führen.

Es zeigen:
- Figur 1: eine Messvorrichtung, die zur Messung einer Körperfunktion eines Patienten auf der Hautoberfläche des Patienten angebracht wird,
- Figur 2: eine mögliche Ausführungsform einer Auswerteschaltung zur Erkennung, ob sich die Messvorrichtung noch in einem ausreichenden Kontakt mit der Hautoberfläche des Patienten befindet,
- Figur 3: mögliche Ausführungsformen von auf einer Unterseite der Messvorrichtung gebildeten oder angebrachten Kontaktflächen,
- Figur 4: eine Messvorrichtung gemäß Figur 1 mit weiteren Details und
- Figur 5: eine Messvorrichtung gemäß Figur 1 und Figur 4 mit einer optionalen lokalen Energiequelle.

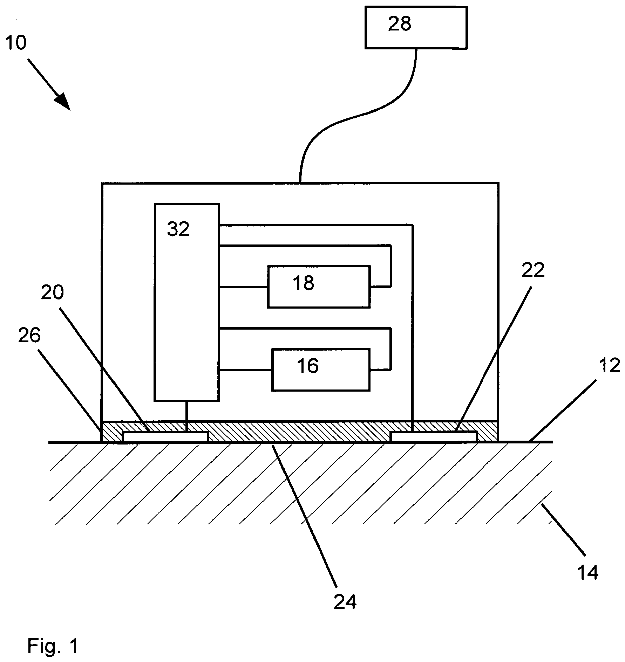

Figur 1 zeigt eine schematisch vereinfachte Darstellung einer Messvorrichtung 10, die zur Messung einer Körperfunktion eines Menschen oder Patienten auf der Oberfläche (Hautoberfläche 12) der Haut 14 eines Menschen oder Patienten anbringbar ist und bei Benutzung angebracht wird. Die Messvorrichtung 10 umfasst einen ersten Sensor 16 und in der dargestellten Ausführungsform einen optionalen zweiten Sensor 18 (einzeln oder zusammen auch als Sensorik bezeichnet) sowie zumindest eine erste Kontaktfläche 20 und zumindest eine zweite Kontaktfläche 22 auf einer zum Kontakt mit der Hautoberfläche 12 vorgesehenen Unterseite 24 der Messvorrichtung 10. Jeder Sensor 16, 18 kann innerhalb der Messvorrichtung 10 so angeordnet sein, dass er sich an der Unterseite 24 der Messvorrichtung 10 befindet und dabei selbst unmittelbar mit der Hautoberfläche 12 in Kontakt steht. Ein Sensor 16, 18, der sich im Innern der Messvorrichtung 10 befindet, steht über die umgebende Messvorrichtung 10 zumindest noch mittelbar mit der Hautoberfläche 12 in Kontakt.

Wie schon eingangs definiert, werden auch hier die zumindest eine erste Kontaktfläche 20 und die zumindest eine zweite Kontaktfläche 22 zur sprachlichen Vereinfachung kurz als erste und zweite Kontaktfläche und zusammen als "die Kontaktflächen" oder "beide Kontaktflächen" bezeichnet. Anstelle des Ausdrucks Menschen oder Patienten wird im Folgenden ebenfalls im Sinne einer sprachlichen Vereinfachung durchgängig der Begriff Patient verwendet. Dieser ist jedoch weit auszulegen, so dass auch nicht in medizinischer Behandlung befindliche Personen umfasst sind, denn der hier und im Folgenden beschriebene Ansatz kommt zum Beispiel auch bei einer Messung einer Körperfunktion eines Sportlers oder dergleichen in Betracht.

Bei der in Figur 1 gezeigten Ausführungsform der Messvorrichtung 10 sind die Kontaktflächen 20, 22 in eine optionale Haftschicht 26 in Form einer dünnen Folie eingebettet oder auf dieser aufgebracht. Diese Haftschicht 26 befindet sich an einer zum Kontakt mit der Hautoberfläche 12 des Patienten vorgesehenen Unterseite 24 der Messvorrichtung 10. Die Folie wirkt als Haftschicht 26, weil sie aufgrund ihrer Materialeigenschaften und Oberflächenstruktur geeignet ist, eine Haftung der Messvorrichtung 10 auf der Hautoberfläche 12 des Patienten aufgrund von Van-der-Waalschen Kräften zu bewirken. Die Folie/Haftschicht 26 ist von der Messvorrichtung 10 als integraler Bestandteil umfasst. Ohne eine solche Haftschicht 26 befinden sich die Kontaktflächen 20, 22 an der sich dann ergebenden Unterseite 24 der Messvorrichtung 10 und für eine Haftung der Messvorrichtung 10 auf der Hautoberfläche 12 wird zum Beispiel ein Kleber oder ein Hydrogel auf die Unterseite 24 aufgetragen. Eine solcher Haftauftrag ist im Gegensatz zu der als Haftschicht 26 fungierenden Folie kein Bestandteil der Messvorrichtung 10 und wird nur bei Bedarf aufgetragen.

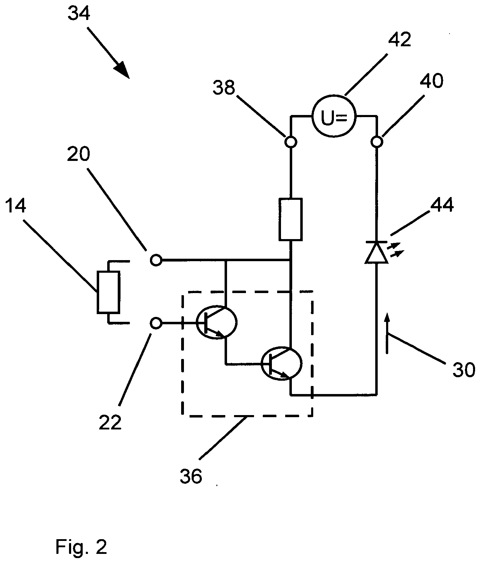

Die Messvorrichtung 10 ist an ein nicht näher dargestelltes und in der Fachterminologie üblicherweise als Monitor bezeichnetes Medizingerät 28 anschließbar und bei Benutzung an ein solches Medizingerät 28 angeschlossen. Die Messvorrichtung 10 umfasst Mittel zum Erfassen eines aufgrund eines jeweils wirksamen elektrischen Widerstands zwischen den Kontaktflächen 20, 22 resultierenden Messstroms 30 (Figur 2) oder zum Erfassen eines elektrischen Widerstands oder einer elektrischen Leitfähigkeit zwischen den Kontaktflächen 20, 22.

Als Mittel zum Erfassen des jeweiligen Messstroms 30, elektrischen Widerstands oder einer elektrischen Leitfähigkeit ist in der Darstellung in Figur 1 schematisch vereinfacht eine Elektronik 32 gezeigt. Die Elektronik 32 umfasst zum Beispiel eine Auswerteschaltung 34 mit einer an sich bekannten sogenannten Darlington-Schaltung 36, wie sie in Figur 2 gezeigt ist.

Anhand der eine solche Darlington-Schaltung 36 umfassenden Auswerteschaltung 34, jedoch ohne Verzicht auf eine weitergehende Allgemeingültigkeit, wird im Folgenden erläutert, wie sich mit der hier vorgeschlagenen Messvorrichtung 10 die Erkennung eines ungenügenden Hautkontakts und damit eine Erhöhung der Verlässlichkeit der mit der Sensorik 16, 18 jeweils erfassten Messwerte und/oder eine Verbesserung der Sicherheit des Patienten erreichen lässt.

Die beiden Kontaktflächen 20, 22 der Messvorrichtung 10 sind in Figur 2 im Sinne einer einfachen Darstellung als Kontaktstellen gezeigt. Der vor diesen Kontaktstellen gezeigte Widerstand entspricht der Haut 14 und der Hautoberfläche 12 des jeweiligen Patienten. Neben den beiden als Ausgängen der Auswerteschaltung 34 fungierenden Kontaktflächen 20, 22 umfasst diese zwei als Eingänge 38, 40 fungierende Kontaktstellen. Hier ist eine externe Gleichspannungsquelle 42 anschließbar. Diese ist geeignet, den Messstrom 30 über die Auswerteschaltung 34 zu treiben. Der Messstrom 30 kann in der Auswerteschaltung 34 allerdings nur fließen, wenn ein elektrisch leitender Kontakt zwischen den Kontaktflächen 20, 22 besteht. Ein solcher Kontakt kann über die Haut 14 und die Hautoberfläche 12 des Patienten hergestellt werden. Dafür ist erforderlich, dass beide Kontaktflächen 20, 22 auf der Hautoberfläche 12 aufliegen. Nur dann fließt zwischen den Kontaktflächen 20, 22 ein (aufgrund des vergleichsweise hohen elektrischen Widerstands eher geringer) elektrischer Strom, der mit der Darlington-Schaltung 36 - oder einer sonst geeigneten Verstärkungsschaltung - so weit verstärkt wird, dass mittels des dann fließenden Messstroms 30 zum Beispiel eine optionale Anzeigeeinrichtung 44, die hier in Form einer LED gezeigt ist, zum Leuchten gebracht werden kann. Eine aktive, leuchtende Anzeigeeinrichtung 44 zeigt also einen Kontakt der Kontaktflächen 20, 22 mit der Hautoberfläche 12 des Patienten an. Eine inaktive Anzeigeeinrichtung 44 zeigt entsprechend einen unterbrochenen oder ungenügenden Kontakt der Kontaktflächen 20, 22 mit der Hautoberfläche 12 an.

Der jeweilige Messstrom 30 zeigt damit an, ob sich die Messvorrichtung 10 noch in Kontakt mit der Hautoberfläche 12 des Patienten befindet und wird entsprechend auch als Kontaktsignal bezeichnet. Ein von der Sensorik 16, 18 (Figur 1) gelieferter Messstrom wird zur Unterscheidung als Sensorsignal bezeichnet.

Indem die Elektronik 32 der Messvorrichtung 10 eine solche oder gleichwirkende Auswerteschaltung 34 umfasst, ist also die Erkennung eines ungenügenden Haut-kontakts und eine Generierung eines entsprechenden Kontaktsignals möglich. Auf Basis einer solchen Erkennungsmöglichkeit ist auch eine Erhöhung der Verlässlichkeit der mit der Messvorrichtung 10 und deren Sensorik 16, 18 jeweils erfassten Messwerte und/oder eine Verbesserung der Sicherheit des Patienten möglich.

Dies ist speziell dann gegeben, wenn von den erfassten Messwerten Behandlungsmaßnähmen abhängen, die von einem jeweiligen Medizingerät 28, an welches die Messvorrichtung 10 angeschlossen ist, automatisch bewirkt werden. Ein solches Medizingerät 28 ist zum Beispiel ein Inkubator für Neugeborene, der als Behandlungsmaßnahme eine Regelung der Umgebungstemperatur für das Neugeborene, also eine Regelung einer Innentemperatur des Inkubators, in Abhängigkeit von dessen Körpertemperatur bewirkt. Falls bei einer Erfassung der Körpertemperatur mittels einer Messvorrichtung 10 dessen Temperaturmesswert (auf Basis des Sensorsignals) nicht mehr verlässlich ist, weil die Messvorrichtung 10 sich nicht mehr in ausreichendem Kontakt mit der Hautoberfläche 12 des Neugeborenen befindet, darf die Temperaturregelung nicht mehr auf Basis eines dann noch generierten Messwerts erfolgen. Die dann vorliegende Fehlersituation muss in geeigneter Art und Weise signalisiert werden. Dies kann mittels einer Anzeigeeinrichtung 44 geschehen. Das Kontaktsignal, der Messstrom 30, oder ein fehlender Messstrom 30 kann jedoch zusätzlich oder alternativ auch automatisch ausgewertet werden, so dass ein nachgeschaltetes Medizingerät 28 oder dergleichen, wie zum Beispiel ein Inkubator, in einen vorgebbaren oder vorgegebenen Zustand versetzt wird und/oder das Medizingerät 28 ein optisches und/oder akustisches Alarmsignal ausgibt.

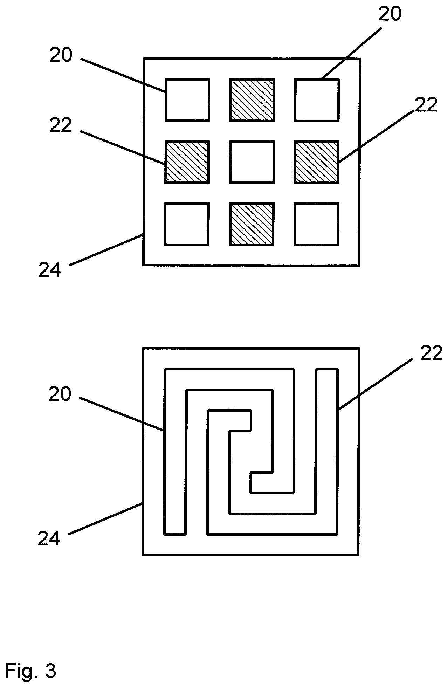

Die Darstellung in Figur 3 zeigt zwei mögliche Ausführungsformen einer Unterseite 24 der Messvorrichtung 10. Im oberen Bereich der Darstellung in Figur 3 ist eine Anordnung der Kontaktflächen 20, 22 in Form eines Rasters gezeigt. Man erkennt dabei, dass die Messvorrichtung 10 auch eine Mehrzahl erster und zweiter Kontaktflächen 20, 22 umfassen kann. Die zweiten Kontaktflächen 22 sind im oberen Bereich der Darstellung zur Unterscheidung von den ersten Kontaktflächen 20 durch eine Schraffur kenntlich gemacht. Im unteren Bereich der Darstellung in Figur 3 ist eine einfache labyrinthische Anordnung der Kontaktflächen 20, 22 gezeigt.

Die Besonderheit einer solchen oder ähnlichen Anordnung und/oder Verteilung der Kontaktflächen 20, 22 besteht darin, dass bei einem Abheben zum Beispiel der bei der rasterartigen Anordnung der Kontaktflächen 20, 22 links oben dargestellten ersten Kontaktfläche 20 der Strom noch von einer der anderen ersten Kontaktflächen 20 über die Haut 14 und die Hautoberfläche 12 zu den zweiten Kontaktflächen 22 fließen kann. Eine nur punktuelle Ablösung der Messvorrichtung 10 von der Hautoberfläche 12, bei der noch verlässliche Messwerte von der jeweiligen Sensorik 16, 18 erhältlich sind, muss also nicht als Fehlersituation ausgewertet werden. Wenn dagegen die Messvorrichtung 10, wie dies bei einer besonders einfachen oder einer im Hinblick auf eine Erkennung eines Ablösens von der Hautoberfläche 12 besonders sensitiven Messvorrichtung 10 ohne Weiteres vorgesehen sein kann, genau eine erste und genau eine zweite Kontaktfläche 20, 22 mit jeweils geringen räumlichen Abmessungen aufweist, führt jedes Abheben entweder der genau einen ersten oder der genau einen zweiten Kontaktfläche 20, 22 zu einer Unterbrechung des Messstroms 30 und damit zu einer automatisch erkennbaren und signalisierbaren Fehlersituation.

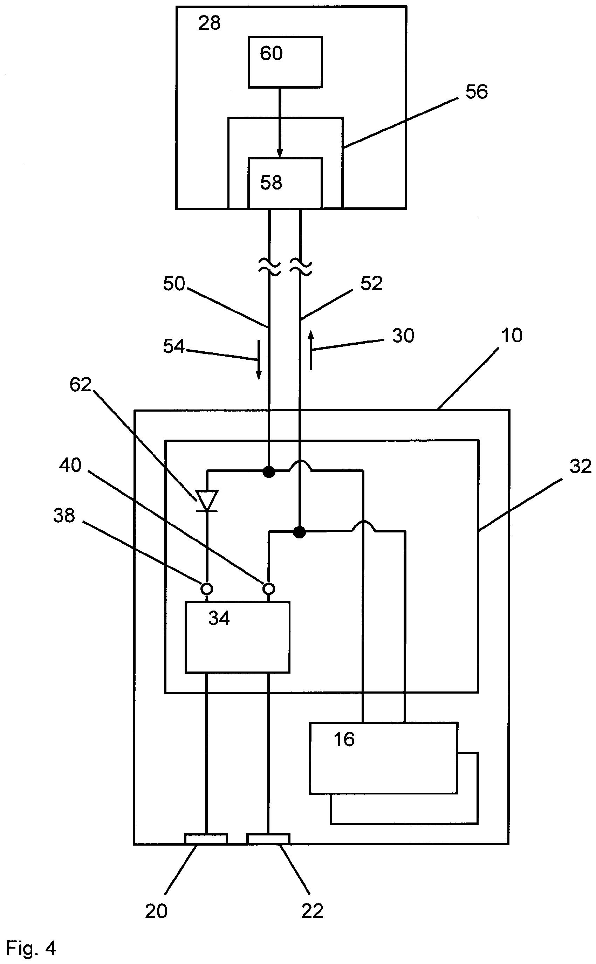

Figur 4 zeigt eine schematisch vereinfachte Darstellung der Messvorrichtung 10 aus Figur 1, wobei die Auswerteschaltung 34 gemäß Figur 2 (oder eine Auswerteschaltung mit einer vergleichbaren Funktionalität) als von der Elektronik 32 umfasst dargestellt ist. Zur Generierung eines jeweiligen Messstroms 30 wird der Messvorrichtung 10 und deren Elektronik 32 von einem Medizingerät 28 über hier genau zwei Zuleitungen 50, 52 ein Speisestrom 54 zugeführt. Die Sensorik 16, 18 und die Kontaktflächen 20, 22 - hier die Sensorik 16, 18 auf der einen Seite und die Kontaktflächen 20, 22 zusammen mit ihrer Auswerteschaltung 34 auf der anderen Seite - sind innerhalb der Messvorrichtung 10 elektrisch parallel geschaltet, so dass der Speisestrom 54 grundsätzlich zur Sensorik 16, 18 und zur Auswerteschaltung 34 gelang.

An das Medizingerät 28 ist üblicherweise eine Mehrzahl der hier beschriebenen Messvorrichtungen 10 oder anderer Messvorrichtungen anschließbar. Der Anschluss einer Messvorrichtung 10 erfolgt an einen Eingangskanal 56 des Medizingeräts 28. Als Bestandteil einer Beschaltung eines solchen Eingangskanals 56 umfasst das Medizingerät 28 eine an sich bekannte und daher nicht näher dargestellte Polwendeschaltung 58, die auf Seiten des Medizingeräts 28 mittels eines Taktgebers 60 getaktet angesteuert wird. Im Ergebnis wird damit der Messvorrichtung 10 der Speisestrom 54 während einer ersten Schaltstellung der Polwendeschaltung 58 über die erste Zuleitung 50 und während einer komplementären zweiten Schaltstellung der Polwendeschaltung 58 über die zweite Zuleitung 52 zugeführt. Damit eine Differenzierung auf Seiten der Messvorrichtung 10 möglich ist, ist entweder in dem Strompfad zu den Kontaktflächen 20, 22 oder in dem Strompfad zur Sensorik 16, 18 ein Stromsperrelement 62, also zum Beispiel eine Diode 62, vorgesehen. Die in Figur 4 gezeigte Diode 62 bewirkt, dass bei einem über die erste Zuleitung 50 zugeführten Speisestrom 54 dieser einerseits zu der Auswerteschaltung 34 und den Kontaktflächen 20, 22 und andererseits zur Sensorik 16, 18 gelangt. Der resultierende, zum Medizingerät 28 zurückfließende Messstrom 30 entspricht demnach der Summe der von der Auswerteschaltung 34 und der Sensorik 16, 18 generierten Messströme 30. Der zum Medizingerät 28 zurückfließende Messstrom 30 ist demnach ein Summensignal von der Auswerteschaltung 34 und der Sensorik 16, 18. Für einen (nach einer Polwendung auf Seiten des Medizingeräts 28) über die zweite Zuleitung 52 zugeführten Speisestrom 54 ist die Diode 62 in Sperrrichtung geschaltet, so dass der resultierende, zum Medizingerät 28 zurückfließende Messstrom 30 nur von der Sensorik 16, 18 und dem dort generierten Messstrom abhängt.

Der Beitrag der Auswerteschaltung 34 zu dem Summensignal ist bekannt (zum Beispiel aufgrund einer maximalen dortigen Stromverstärkung), so dass der dem Summensignal zugrunde liegende Messstrom 30 mit einem vorgegebenen Schwellwert verglichen werden kann, um zu ermitteln, ob sich die Messvorrichtung 10 noch in einem ausreichenden Kontakt mit der Hautoberfläche 12 befindet. Wenn ein solcher Kontakt nicht mehr gegeben ist und das Summensignal also keinen Beitrag aufgrund des Kontaktsignals enthält, liegt der Messstrom 30 unter einem geeignet gewählten Schwellwert. Der Schwellwert wird dazu anhand des normalerweise von dem Kontaktsignal erwarteten Beitrags zu dem Summensignal gewählt, zum Beispiel in Form eines um 5%, 10% oder dergleichen verringerten Wertes.

Der von der Messvorrichtung 10 mit der dortigen Sensorik 16, 18 aufgenommene Messwert kann in den entsprechenden Taktphasen direkt anhand des jeweils zurückgelieferten Messstroms 30 ausgewertet werden. Dafür kommt in Betracht, dass anhand des Messstroms 30 auf Seiten des Medizingeräts 28 mit einem Digital/Analog-Wandler ein mittels einer (nicht gezeigten) Elektronik des Medizingeräts 28 verarbeitbarer Digitalwert gebildet wird, der zum Beispiel auf einem Bildschirm oder dergleichen des Medizingeräts 28 angezeigt und/oder in einem Speicher des Medizingeräts 28 protokolliert werden kann.

Bei einer besonderen, nicht gezeigten Ausführungsform der Messvorrichtung 10 befindet sich sowohl in dem Strompfad zu den Kontaktflächen 20, 22 wie auch in dem Strompfad zur Sensorik 16, 18 jeweils ein Stromsperrelement 62, also zum Beispiel jeweils eine Diode 62, und zwar entweder im Anschluss an die erste Zuleitung 50 mit antiparallelen Sperrrichtungen oder jeweils im Anschluss an die erste Zuleitung 50 und die zweite Zuleitung 52 mit parallelen Sperrrichtungen. Dann wird bei einer getakteten alternierenden Bestromung der Messvorrichtung 10 während jeder Taktphase nur entweder das Sensorsignal oder das Kontaktsignal generiert und der zurückgelieferte Messstrom 30 ist direkt ein Maß für das jeweilige Signal.

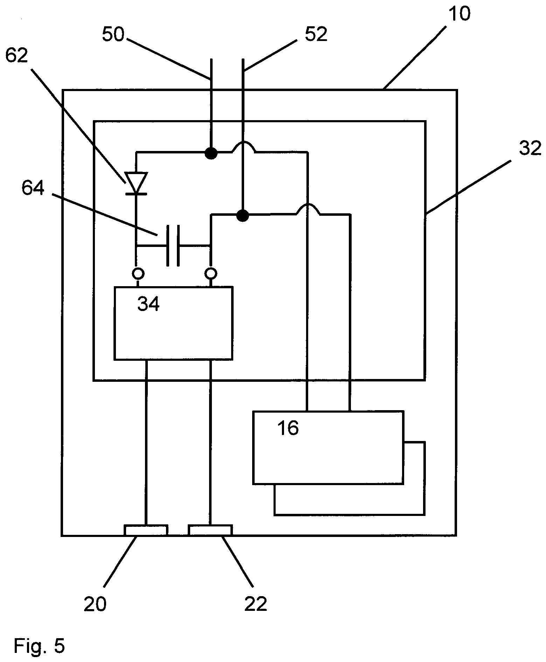

Die Darstellung in Figur 5 zeigt weitere, allerdings optionale Details der Messvorrichtung 10. Danach umfasst die Messvorrichtung 10 eine lokale Energiequelle 64, zum Beispiel in Form eines Kondensators 64 oder dergleichen. Der Kondensator 64 wird mittels des Speisestroms 54 geladen. Bei einer getakteten Bestromung der Messvorrichtung 10 wirkt der Kondensator 64 wie die in Figur 2 gezeigte Gleichspannungsquelle 42 und ermöglicht zum Beispiel eine Energieversorgung einer Anzeigeeinrichtung 44 auch für die kurzen Zeitspannen der getakteten Bestromung, während derer die Diode 62 den Speisestrom 54 sperrt. Als zusätzliche oder alternative Möglichkeit zur Realisierung einer lokalen Energiequelle 64 kommt ein Thermoelement oder ein Peltier-Element (PT-Element) in Betracht (nicht gezeigt). Bei einer solchen lokalen Energiequelle 64 lässt sich eine üblicherweise anzunehmende Temperaturdifferenz zwischen einer Temperatur auf der Hautoberfläche 12 des Patienten und einer Umgebungstemperatur zur Speisung der Auswerteschaltung 34 und zur Energieversorgung der Anzeigeeinrichtung 44 verwenden.

Die in Figur 2 gezeigte Auswerteschaltung 34 stellt nur eine Realisierungsmöglichkeit für Mittel zum Erfassen eines aufgrund eines jeweils wirksamen elektrischen Widerstands zwischen den beiden Kontaktflächen 20, 22 resultierenden Messstroms 30 dar. Die gezeigte Auswerteschaltung 34 ist dabei eine besonders einfache Realisierungsmöglichkeit. Genauso kommt eine Realisierung solcher Mittel zum Erfassen eines jeweils resultierenden Messstroms 30 in Form eines ASICs oder dergleichen (nicht gezeigt) in Betracht. Dann ist zum Beispiel eine Ansteuerung einer Anzeigeeinrichtung 44 in Form einer LED oder dergleichen oder mehrerer LEDs möglich, die dem Patienten und/oder dem medizinischen Personal den jeweiligen Status des Kontaktsignals durch Farbumschlag oder alternierende Aktivierung jeweils genau einer Anzeigeeinrichtung 44 anzeigt. Ein solcher ASIC oder dergleichen lässt sich mit dem Speisestrom 54 und ansonsten zumindest kurzzeitig aus einer lokalen Energiequelle 64 - wie oben beschrieben - mit ausreichender elektrischer Energie versorgen, denn ASICs oder andere Formen integrierter Halbleiterschaltungen haben bekanntlich nur eine sehr geringe Leistungsaufnahme.

Einzelne im Vordergrund stehende Aspekte der hier eingereichten Beschreibung lassen sich damit kurz wie folgt zusammenfassen: Angegeben werden eine Messvorrichtung 10 mit mindestens einem Sensor 16, 18, die zur Erfassung eines Messwerts mittels des Sensors 16, 18 auf der Hautoberfläche 12 eines Patienten anbringbar ist und sich dadurch auszeichnet, dass sie zwei Kontaktflächen 20, 22 auf einer zum Kontakt mit der Hautoberfläche 12 des Patienten vorgesehenen Unterseite 24 sowie Mittel 32, 34 zum Erfassen eines aufgrund eines jeweils wirksamen elektrischen Widerstands zwischen den Kontaktflächen 20, 22 resultierenden Messstroms 30 aufweist, sowie ein Verfahren zum Betrieb einer solchen Messvorrichtung 10 und ein System mit einer solchen Messvorrichtung 10.

### BEZUGSZEICHENLISTE

- 10: Messvorrichtung
- 12: Hautoberfläche
- 14: Haut
- 16: (erster) Sensor
- 18: (zweiter) Sensor
- 20: (erste) Kontaktfläche
- 22: (zweite) Kontaktfläche
- 24: Unterseite (der Messvorrichtung)
- 26: Haftschicht
- 28: Medizingerät
- 30: Messstrom
- 32: Elektronik
- 34: Auswerteschaltung
- 36: Darlington-Schaltung
- 38: Eingang (der Auswerteschaltung)
- 40: Eingang (der Auswerteschaltung)
- 42: externe Gleichspannungsquelle
- 44: Anzeigeeinrichtung
- 50: (erste) Zuleitung (für die Messvorrichtung)
- 52: (zweite) Zuleitung (für die Messvorrichtung)
- 54: Speisestrom
- 56: Eingangskanal (des Medizingeräts)
- 58: Polwendeschaltung
- 60: Taktgeber
- 62: Stromsperrelement, insbesondere Diode
- 64: lokale Energiequelle, insbesondere Kondensator

## Patentansprüche

1. Messvorrichtung (10) mit mindestens einem Sensor (16, 18), wobei die Messvorrichtung (10) zur Erfassung eines Messwerts mittels des Sensors (16, 18) auf der Hautoberfläche (12) eines Patienten anbringbar ist,
**gekennzeichnet durch**
zumindest eine erste und zumindest eine zweite Kontaktfläche (20, 22) auf einer zum Kontakt mit der Hautoberfläche (12) des Patienten vorgesehenen Unterseite (24) der Messvorrichtung (10) sowie
Mittel (32, 34) zum Erfassen eines aufgrund eines jeweils wirksamen elektrischen Widerstands zwischen der zumindest einen ersten und zweiten Kontaktfläche (20, 22) resultierenden Messstroms (30), der ein Kontaktsignal umfasst, wobei der oder jeder Sensor (16, 18) und die zumindest eine erste und zweite Kontaktfläche (20, 22) elektrisch parallel geschaltet sind und wobei sich entweder in einem Strompfad zu dem oder jedem Sensor (16, 18) oder in einem Strompfad zu der zumindest einen ersten und zweiten Kontaktfläche (20, 22) ein Stromsperrelement (62), insbesondere eine Diode (62), befindet.

2. Messvorrichtung (10) nach Anspruch 1, die neben dem Sensor (16) einen weiteren artgleichen Sensor (18) umfasst.

3. Messvorrichtung (10) nach Anspruch 1 oder 2, mit in Form eines Rasters oder labyrinthisch auf der Unterseite (24) der Messvorrichtung (10) angeordneten Kontaktflächen (20, 22).

4. Messvorrichtung (10) nach einem der vorangehenden Ansprüche, mit einer in Abhängigkeit von dem erfassten elektrischen Widerstand oder der erfassten elektrischen Leitfähigkeit zwischen der zumindest einen ersten und zweiten Kontaktfläche (20, 22) ansteuerbaren Anzeigeeinrichtung (44).

5. Messvorrichtung (10) nach Anspruch 4, mit einer lokalen Energiequelle (64) zur Energieversorgung zumindest der Anzeigeeinrichtung (44).

6. Messvorrichtung (10) nach einem der vorangehenden Ansprüche, mit einer von der Messvorrichtung (10) umfassten Haftschicht (26) auf einer zum Kontakt mit der Hautoberfläche (12) des Patienten vorgesehenen Unterseite (24) der Messvorrichtung (10), die geeignet ist, eine Haftung der Messvorrichtung (10) auf der Hautoberfläche (12) des Patienten aufgrund von Van-der-Waalschen Kräften zu bewirken, wobei die zumindest eine erste und zweite Kontaktfläche (20, 22) an einer zum Kontakt mit der Hautoberfläche (12) des Patienten vorgesehenen Oberfläche der Haftschicht (26) in diese eingebettet sind oder auf dieser aufgebacht sind.

7. Messvorrichtung (10) nach einem der vorangehenden Ansprüche, wobei der oder jeder Sensor (16, 18) ein Temperatursensor ist und die Messvorrichtung (10) als Temperaturmessvorrichtung fungiert.

8. Verfahren zum Betrieb einer Messvorrichtung (10) nach einem der vorangehenden Ansprüche, wobei der Messvorrichtung (10) ein Speisestrom (54) alternierend über eine erste Zuleitung (50) oder eine zweite Zuleitung (52) zugeführt wird.

9. Verfahren nach Anspruch 8 zum Betrieb einer Messvorrichtung (10) mit einer lokalen Energiequelle (64), wobei der Speisestrom (54) für ein Aufladen der Energiequelle (64) verwendet wird.

10. Verfahren nach Anspruch 8 zum Betrieb einer Messvorrichtung (10) mit einer lokalen Energiequelle (64), wobei mittels eines elektrothermischen Wandlers eine im Bereich der Messvorrichtung (10) herrschende Temperaturdifferenz für ein Aufladen der Energiequelle (64) verwendet wird.

11. System mit einem Medizingerät (28) und zumindest einer mittels einer ersten Zuleitung (50) und einer zweiten Zuleitung (52) an das Medizingerät (28) angeschlossenen Messvorrichtung (10) nach einem der Ansprüche 1 bis 7, wobei der Messvorrichtung (10) mittels des Medizingeräts (28) alternierend über die erste und die zweite Zuleitung (50, 52) ein Speisestrom (54) zuführbar ist und dem Medizingerät (28) durch die Messvorrichtung (10) über eine jeweils komplementäre Zuleitung (52, 50) ein Messstrom (30), der ein Summensignal aus einem Sensorsignal und dem Kontaktsignal aufweist, zuführbar ist, der mittels des Medizingeräts (28) im Betrieb auswertbar ist.

## Claims

1. A measuring device (10) having at least one sensor (16, 18), wherein the measuring device (10) can be placed on the skin surface (12) of a patient in order to detect a measured value by means of the sensor (16, 18),
**characterised by**
at least one first and at least one second contact surface (20, 22) on an underside (24) of the measuring device (10) that is provided for contact with the skin surface (12) of the patient, and also
means (32, 34) for detecting a measuring current (30) that results on account of an electrical resistance effective in each case between the at least one first and second contact surface (20, 22) and comprises a contact signal, wherein the or each sensor (16, 18) and the at least one first and second contact surface (20, 22) are connected electrically in parallel, and wherein a power cut-off element (62), in particular a diode (62), is located either in a current path to the or each sensor (16, 18) or in a current path to the at least one first and second contact surface (20, 22).

2. A measuring device (10) according to claim 1 which in addition to the sensor (16) comprises a further sensor (18) of the same type.

3. A measuring device (10) according to claim 1 or 2 having contact surfaces (20, 22) arranged in the form of a grid or in a labyrinthine pattern on the underside (24) of the measuring device (10).

4. A measuring device (10) according to one of the preceding claims having a display device (44) which can be activated as a function of the detected electrical resistance or the detected electrical conductivity between the at least one first and second contact surface (20, 22).

5. A measuring device (10) according to claim 4 having a local energy source (64) for supplying energy at least to the display device (44).

6. A measuring device (10) according to one of the preceding claims having an adhesive layer (26), comprised by the measuring device (10), on an underside (24) of the measuring device (10) provided for contact with the skin surface (12) of the patient, that is suitable for effecting adhesion of the measuring device (10) to the skin surface (12) of the patient on the basis of van der Waals forces, wherein the at least one first and second contact surface (20, 22) are embedded in or applied to a surface of the adhesive layer (26) that is provided for contact with the skin surface (12) of the patient.

7. A measuring device (10) according to one of the preceding claims, wherein the or each sensor (16, 18) is a temperature sensor, and the measuring device (10) functions as a temperature-measuring device.

8. A method for operating a measuring device (10) according to one of the preceding claims, wherein a feed current (54) is fed to the measuring device (10) alternately by way of a first feed line (50) or a second feed line (52).

9. A method according to claim 8 for operating a measuring device (10) with a local energy source (64), wherein the feed current (54) is used to charge the energy source (64).

10. A method according to claim 8 for operating a measuring device (10) with a local energy source (64), wherein a temperature difference prevailing in the region of the measuring device (10) is used to charge the energy source (64) by means of an electrothermal converter.

11. A system having a medical device (28) and at least one measuring device (10) according to one of claims 1 to 7 that is connected to the medical device (28) by means of a first feed line (50) and a second feed line (52), wherein a feed current (54) can be fed to the measuring device (10) by means of the medical device (28) alternately by way of the first and the second feed line (50, 52), and there can be fed to the medical device (28) by means of the measuring device (10) by way of a respective complementary feed line (52, 50) a measuring current (30) that has a sum signal of a sensor signal and the contact signal and can be evaluated by means of the medical device (28) during operation.

## Revendications

1. Dispositif de mesure (10) comprenant au moins un capteur (16, 18), le dispositif de mesure (10) pouvant être appliqué sur la surface cutanée (12) d'un patient en vue de détecter une valeur mesurée au moyen du capteur (16, 18),
**caractérisé par**
au moins une première et au moins une deuxième surface de contact (20, 22) sur un côté inférieur (24) du dispositif de mesure (10) conçu pour entrer en contact avec la surface cutanée (12) du patient ainsi que des moyens (32, 34) destinés à détecter un courant de mesure (30) produit en raison d'une résistance électrique respectivement active entre les au moins une première et deuxième surfaces de contact (20, 22), lequel comprend un signal de contact, le ou chaque capteur (16, 18) et les au moins une première et deuxième surfaces de contact (20, 22) étant branchés électriquement en parallèle et un élément de blocage de courant (62), notamment une diode (62), se trouvant soit dans un trajet de courant vers le ou chaque capteur (16, 18), soit dans un trajet de courant vers les au moins une première et deuxième surfaces de contact (20, 22).

2. Dispositif de mesure (10) selon la revendication 1, lequel comporte un capteur (18) supplémentaire du même type en plus du capteur (16).

3. Dispositif de mesure (10) selon la revendication 1 ou 2, comprenant des surfaces de contact (20, 22) disposées sur le côté inférieur (24) du dispositif de mesure (10) sous la forme d'une grille ou d'un labyrinthe.

4. Dispositif de mesure (10) selon l'une des revendications précédentes, comprenant un appareil d'affichage (44) qui peut être commandé en fonction de la résistance électrique détectée ou de la conductivité électrique détectée entre les au moins une première et deuxième surfaces de contact (20, 22).

5. Dispositif de mesure (10) selon la revendication 4, comprenant une source d'énergie locale (64) destinée à alimenter en énergie au moins l'appareil d'affichage (44).

6. Dispositif de mesure (10) selon l'une des revendications précédentes, comprenant une couche d'adhérence (26) englobée par le dispositif de mesure (10) sur un côté inférieur (24) du dispositif de mesure (10) conçu pour entrer en contact avec la surface cutanée (12) du patient, laquelle est adaptée pour produire une adhérence du dispositif de mesure (10) sur la surface cutanée (12) du patient en raison de forces de Van der Waals, les au moins une première et deuxième surfaces de contact (20, 22) étant enrobées dans la couche d'adhérence (26) ou appliquées sur celle-ci au niveau d'une surface de celle-ci conçue pour entrer en contact avec la surface cutanée (12) du patient.

7. Dispositif de mesure (10) selon l'une des revendications précédentes, le ou chaque capteur (16, 18) étant un capteur de température et le dispositif de mesure (10) faisant office de dispositif de mesure de la température.

8. Procédé pour faire fonctionner un dispositif de mesure (10) selon l'une des revendications précédentes, un courant d'alimentation (54) étant acheminé au dispositif de mesure (10) en alternance par le biais d'une première ligne d'arrivée (50) ou d'une deuxième ligne d'arrivée (52).

9. Procédé selon la revendication 8 pour faire fonctionner un dispositif de mesure (10) comprenant une source d'énergie locale (64), le courant d'alimentation (54) étant utilisé pour une charge de la source d'énergie (64).

10. Procédé selon la revendication 8 pour faire fonctionner un dispositif de mesure (10) comprenant une source d'énergie locale (64), une différence de température qui règne dans la zone du dispositif de mesure (10) étant utilisée au moyen d'un convertisseur électrothermique pour une charge de la source d'énergie (64).

11. Système comprenant un appareil médical (28) et au moins un dispositif de mesure (10) selon l'une des revendications 1 à 7 raccordé à l'appareil médical (28) au moyen d'une première ligne d'arrivée (50) et d'une deuxième ligne d'arrivée (52), un courant d'alimentation (54) pouvant être acheminé au dispositif de mesure (10) au moyen de l'appareil médical (28) en alternance par le biais de la première et de la deuxième ligne d'arrivée (50, 52) et un courant de mesure (30), lequel possède un signal total composé d'un signal de capteur et du signal de contact, pouvant être acheminé à l'appareil médical (28) par le dispositif de mesure (10) par le biais d'une ligne d'arrivée (52, 50) respectivement complémentaire, lequel peut être interprété par l'appareil médical (28) en fonctionnement.
